(19) Europäisches Patentamt
European Patent Office
Office européen des brevets

(11) **EP 4 417 208 A2**

(12) **EUROPEAN PATENT APPLICATION**

(43) Date of publication:
**21.08.2024 Bulletin 2024/34**

(21) Application number: **24171536.6**

(22) Date of filing: **17.07.2016**

(51) International Patent Classification (IPC):
*A61K 31/728* $^{(2006.01)}$

(52) Cooperative Patent Classification (CPC):
(C-Sets available)
**A61K 9/0048; A61K 31/728; A61K 33/14;
A61K 45/06** (Cont.)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.07.2015 US 201514802460**

(62) Document number(s) of the earlier application(s) in
accordance with Art. 76 EPC:
**16740965.5 / 3 324 934**

(71) Applicant: **i.com Medical GmbH
81241 München (DE)**

(72) Inventor: **Müller-Lierheim, Wolfgang G.K.
81241 München (DE)**

(74) Representative: **Reichert & Lindner
Partnerschaft Patentanwälte
Kaflerstrasse 15
81241 München (DE)**

Remarks:
This application was filed on 22.04.2024 as a
divisional application to the application mentioned
under INID code 62.

(54) **TEAR SUBSTITUTE, FLUID FOR BEING USED AS A TEAR SUBSTITUTE, AND METHOD FOR PRODUCING A TEAR SUBSTITUTE**

(57)     The present invention concerns a tear substitute containing hyaluronan with an intrinsic viscosity $[\eta] > 2.5$ m$^3$/kg and a concentration of < 0.2 % w/v. The invention is also to be expressed as a fluid with the aforementioned specifications which fluid being used as a tear substitute.

Furthermore, the present invention provides for a method of producing a tear substitute, comprising the use of hyaluronan with an intrinsic viscosity $[\eta] > 2.5$ m$^3$/kg and a concentration of < 0.2 % w/v.

EP 4 417 208 A2

(52) Cooperative Patent Classification (CPC): (Cont.)

C-Sets
**A61K 31/728, A61K 2300/00;**
**A61K 33/14, A61K 2300/00**

**Description**

**FIELD OF THE INVENTION**

The present invention concerns a tear substitute, usually also called artificial tears or lubricant eye drops, for the treatment of ocular surface disease like dry eye disease, a fluid to be used as a tear substitute, as well as a method for producing a tear substitute.

**BACKGROUND OF THE INVENTION**

Tear substitutes play an essential role in the treatment of ocular surface disease. As a lubricant they are intended to minimize the friction between lid and corneal epithelium during blinking. Their water binding capacity contributes to the hydration of irritated eyes.

For the physiological function of natural human tears it is characteristic that they exhibit high viscosity in the absence of shear stress as typically 65 mPa·s in the open eye, and low viscosity during blinking as typically 10 mPa·s. This ensures high stability of the tear film in the open eye and low shear stress on the corneal epithelium during blinking. However, the flow characteristics of most of the currently available tear substitutes does not mimic the rheology of human tears. Most eye drops exhibit an almost Newtonian flow characteristics, i.e. they have either low or high viscosity both in the open eye and during blinking.

Over the last 15 years numerous hyaluronan (HA) -based tear substitutes have become available, claiming viscoelastic flow characteristics and long persistence in the eye. Measurements of the flow characteristics of more than 40 different HA-based tear substitute brands resulted in that those containing low HA concentration act like Newtonian fluids with low viscosity whereas those with high HA concentration (gels) have high viscosity during blinking causing blurring and excess shear stress on the corneal epithelium, and nevertheless have lower viscosity than natural tears in healthy eyes between blinking.

Only HA-based tear substitutes utilizing very high average molecular mass HA ($\approx$ 3 MDa) in approximately 0.15 percent concentration can mimic the flow characteristics of human tears. The relation between average molecular mass and intrinsic viscosity $[\eta]$ in $m^3/kg$ is given through the Mark-Houwink equation

$$[\eta] = k \cdot (M_{rm})^a$$

with $M_{rm}$ being the average molecular mass in MDa and the coefficients

$$k = 1.3327 \cdot 10^{-4}$$

and

$$a = 0.6691$$

which values for k and a having been found as most predictive.

Another important aspect is the biochemical function of HA. HA is the most important component of the extracellular matrix of multilayer epithelia without blood supply, like the corneal epithelium. HA organizes the extracellular matrix, provides water storage and retention, is responsible for the diffusion of nutrients and metabolic products, controls keratinocyte proliferation and differentiation, is an efficient radical scavenger in cases of UV exposure, infection, oxidative stress and inflammatory processes with tissue necrosis, and influences the migration of epithelial cells during wound healing. The average molecular mass of HA in the extracellular matrix of healthy epithelia is about 3 to 4 MDa. In case of increased HA degradation, e.g. due to inflammatory processes, HA exhibits an average molecular mass dependent signal function. While high average molecular mass HA acts anti-angiogenically and immunosuppressively, small to medium size HA molecules induce inflammatory factors and stimulate immunity and angiogenesis. The binding of free HA to cell membrane receptors protects the cells from lymphocytic and macrophagic attack.

The half-life of HA in the epithelial extracellular matrix is only about 24 hours. Due to intrinsic aging, commencing from the fifth decade of life the amount of freely available and extractible HA in the intercellular spaces decreases rapidly. This is the main reason for the loss of hydration and thickness of the epidermis in older people, suggesting that HA may also play an essential role in age-related dry eye.

US 2004/0013729 A1 discloses to use methylcellulose for increasing the time available for agents to contact the cornea. Although such methylcellulose in US 2004/0013729 A1 is named a viscoelastic polymer, this is against the true meaning of viscoelasticity because methylcellulose does not have viscoelastic behavior but largely acts like a Newtonian fluid.

**SUMMARY** OF THE INVENTION

The objective of the invention is to provide for tear substitute for improved treatment of ocular surface disease like dry eye disease.

This objective is reached with tear substitute according to claim 1. Further advantageous and preferred embodiments are given in the dependent claims and through combinations thereof.

According to the present invention, a tear substitute comprises hyaluronan with a hyaluronan intrinsic viscosity $[\eta] > 2.5$ m$^3$/kg and a concentration of < 0.2 % w/v.

It is also contemplated by the present invention that the tear substitute contains hyaluronan WITH intrinsic viscosity $[\eta] \geq 2.9$ m$^3$/kg.

According to the invention, the tear substitute may further comprise only substances naturally present in the human eye plus eventually substances which are pharmacologically, metabolically, immunologically and/or anti-microbial effective, or being preservative-free.

Instead, the tear substitute may further comprise glycosaminoglycans, electrolytes, and buffers. The tear substitute may further provide that the electrolytes comprise sodium chloride, and/or the buffers comprise a phosphate buffer in concentration $\leq 1.45$ mmol/l or trometamol.

As a further alterative of the invention, the tear substitute may comprise $\leq 2$ % w/v of substances which are pharmacologically, metabolically, immunologically and/or anti-microbial effective. A further embodiment of the invention provides that the tear substitute comprises $\leq 0.1$ % w/v of substances which are pharmacologically, metabolically, immunologically and/or anti-microbial effective. An even further embodiment of the invention provides that the tear substitute comprises $\leq 0.01$ % w/v substances which are pharmacologically, metabolically, immunologically and/or anti-microbial effective.

Also according to the invention, a fluid comprises hyaluronan with an intrinsic hyaluronan viscosity $[\eta] > 2.5$ m$^3$/kg and a concentration of < 0.2 % w/v, characterized in that said fluid being used as a tear substitute.

It is also contemplated by the present invention that the fluid has the hyaluronan intrinsic viscosity $[\eta] \geq 2.9$ m$^3$/kg.

According to the invention, the fluid may further comprise only substances naturally present in a human eye; and substances which are pharmacologically, metabolically, immunologically and/or anti-microbial effective, or being preservative-free.

Alternatively, the fluid may further comprise glycosaminoglycans, electrolytes, and buffers. Such a fluid may further provide that the electrolytes comprise sodium chloride, and/or the buffers comprise a phosphate buffer in concentration $\leq 1.45$ mmol/l or trometamol.

In a further alternative of the invention, the fluid may comprise $\leq 2$ % w/v substances which are pharmacologically, metabolically, immunologically and/or anti-microbial effective. A further embodiment of the invention provides that the fluid comprises $\leq 0.1$ % w/v substances which are pharmacologically, metabolically, immunologically and/or anti-microbial effective. A further embodiment of the invention provides that the fluid comprises $\leq 0.01$ % w/v substances which are pharmacologically, metabolically, immunologically and/or anti-microbial effective.

A method of producing a tear substitute may comprise a step of using hyaluronan with a hyaluronan intrinsic viscosity $[\eta] > 2.5$ m$^3$/kg and a concentration of < 0.2 % w/v. The method may further provide that the hyaluronan intrinsic viscosity $[\eta] \geq 2.9$ m$^3$/kg.

According to the method, the viscosity-increasing substances are preferably added towards or during or as a final step. It is further preferred that a step of mixing is carried out so as to reach or until reaching a homogeneous mixture. The method may also contemplate a step of providing as a basis initially purified water or water for injection. The method may further contemplate first adding electrolytes, buffers and substances which are not increasing the viscosity to the purified water or water for injection.

Advantageously, for all above formulations of the invention the relation between average molecular mass and intrinsic viscosity $[\eta]$ in m$^3$/kg is given through the Mark-Houwink equation

$$[\eta] = k \cdot (M_{rm})^a$$

with $M_{rm}$ being the average molecular mass in MDa and the coefficients

$$k = 1.3327 \cdot 10^{-4}$$

and

$$a = 0.6691$$

which values for k and a having been found as most predictive.

Further for disclosure purposes the present invention is also a tear substitute that comprises hyaluronan and is characterized by a viscoelastic flow characteristics with the zero shear viscosity being $\geq$ 50 mPa·s, and the viscosity at 1000 s$^{-1}$ shear rate being $\leq$ 12 mPa·s. According to an alternative aspect to be also disclosed the invention is a fluid that comprises hyaluronan and having a viscoelastic flow characteristics with the zero shear viscosity being $\geq$ 50 mPa·s, and the viscosity at 1000 s$^{-1}$ shear rate being $\leq$ 12 mPa·s, characterized in that said fluid being used as a tear substitute. Further for disclosure purposes the present invention also is a method of producing a tear substitute and comprises a step of using viscosity-increasing substances for reaching a viscoelastic flow characteristics with the zero shear viscosity being $\geq$ 50 mPa·s, and the viscosity at 1000 s$^{-1}$ shear rate being $\leq$ 12 mPa·s.

## DETAILED DESCRIPTION OF THE PREFERRED EMBODIMENTS

In the following the invention is explained by way of examples only.

According to a first aspect of the invention there is provided a tear substitute containing hyaluronan with an intrinsic viscosity $[\eta]$ > 2.5 m$^3$/kg and a concentration of < 0.2 % w/v. Preferably with the hyaluronan intrinsic viscosity $[\eta] \geq$ 2.9 m$^3$/kg.

The aforementioned first aspect is also fulfilled by a fluid, containing hyaluronan with an intrinsic viscosity $[\eta]$ > 2.5 m$^3$/kg and a concentration of < 0.2 % w/v, wherein said fluid being used as a tear substitute. Preferably with the hyaluronan intrinsic viscosity $[\eta] \geq$ 2.9 m$^3$/kg.

Both the tear substitute and the fluid of the first aspect preferably being at least essentially mucin-free or in other words having a mucin concentration of < 0,3 % w/v. This means that the flow behaviour or properties are essentially reached or adjusted by hyaluronan and not by mucin naturally present in the tear fluid and mainly responsible for the flow behavior thereof.

The relation between average molecular mass and intrinsic viscosity $[\eta]$ in m$^3$/kg advantageously is given through the Mark-Houwink equation

$$[\eta] = k \cdot (M_{rm})^a$$

with $M_{rm}$ being the average molecular mass in MDa and the coefficients

$$k = 1.3327 \cdot 10^{-4}$$

and

$$a = 0.6691$$

which values for k and a having been found as most predictive.

Also, within the scope of said first aspect of the present invention there is a method of producing a tear substitute, comprising the use of hyaluronan with an intrinsic viscosity $[\eta]$ > 2.5 m$^3$/kg and a concentration of < 0.2 % w/v. Mucin concentration if any is less than 0.3 % w/v. Preferably with the hyaluronan intrinsic viscosity $[\eta] \geq$ 2.9 m$^3$/kg.

Next, for disclosure purposes according to a second aspect of the invention there is provided tear substitute containing hyaluronan and having a viscoelastic flow characteristics with the zero shear viscosity being $\geq$ 50 mPa·s, and the viscosity at 1000 s$^{-1}$ shear rate being $\leq$ 12 mPa·s.

The intrinsic viscosity of sodium hyaluronate is determined in accordance to Ph.Eur. The zero shear viscosity is determined as the steady shear plateau viscosity at vanishing shear rate. For highly viscous formulations, measurement with a controlled stress rheometer is as defined in Ph.Eur. which is well known to a man skilled in the art.

The aforementioned second aspect also is fulfilled by a fluid, which contains hyaluronan and has a viscoelastic flow characteristics with the zero shear viscosity being $\geq$ 50 mPa·s, and the shear viscosity at 1000 s$^{-1}$ shear rate being $\leq$ 12 mPa·s, wherein said fluid being used as tear substitute.

Both the tear substitute and the fluid of the second aspect preferably being at least essentially mucin-free or in other words having a mucin concentration of < 0.3 % w/v. This means that the flow behaviour or properties essentially is reached or adjusted by hyaluronan and not by mucin naturally present in the tear fluid and mainly responsible for the flow behavior thereof.

Furthermore, within the scope of said second aspect of the present invention there also is a method of producing a tear substitute, comprising the use of substances for reaching a viscoelastic flow characteristics with the zero shear viscosity being $\geq$ 50 mPa·s, and the viscosity at 1000 s$^{-1}$ shear rate being $\leq$ 12 mPa·s. Mucin concentration if any is less than 0.3 % w/v.

In the following further preferred and advantageous further developments to be used with the aforementioned first and second aspects of the invention are explained.

Preferably there are contained or used only substances which are naturally present in the human eye plus eventually substances which are pharmacologically, metabolically, immunologically and/or anti-microbial effective.

It is furthermore preferred that there are contained or used glycosaminoglycans, electrolytes, and buffers. Especially the electrolytes include sodium chloride, and/or the buffers include a phosphate buffer in concentration $\leq$ 1.45 mmol/l or trometamol.

An even further preferred embodiment is the content or use of $\leq$ 2 % w/v, especially $\leq$ 0.1 % w/v, and especially preferred $\leq$ 0.01 % w/v substances which are pharmacologically, metabolically, immunologically and/or anti-microbial effective.

With regard to the method within the scope of the present invention it is further preferred, that substances which are increasing the viscosity are added towards or during or as a final step.

According to another preferred embodiment of the method of the present invention, mixing is carried out so as to reach or until reaching a homogeneous mixture. As an alternative or in addition it is preferred to initially provide for purified water or water for injection as a basis, and then, especially, electrolytes, buffers and substances which are not increasing the viscosity are added at first to the purified water or water for injection.

For comparison with tear substitutes, artificial tears or lubricant eye drops having other specifications, tests of the tear substitute having the specifications in the below table were made with patients with ocular surface disease and achieved excellent and the best results:

| Characteristic | Specification | Test Method |
|---|---|---|
| appearance | clear and colorless solution, free from visible impurities | Ph.Eur. |
| pH value | 6.8 - 7.6 | Ph.Eur. |
| osmolality | 240 - 330 mosmol/kg | Ph.Eur. |
| HA concentration | 0.10 - 0.19 % w/v | Ph.Eur. |
| NaCl concentration | 7.6 - 10.5 g/l | Ph.Eur. |
| sterility | sterile | Ph.Eur. |
| Phosphate concentration | 1.0 - 1.4 mmol/l | Ph.Eur. |

The invention is described only exemplarily by the embodiments in the description and drawings and is not limited thereto but rather includes all variations, modifications, substitutions, and combinations the expert may take from the complete documents of this application under consideration of and/or combination with his specific knowledge.

## Claims

1. Tear substitute containing hyaluronan with an intrinsic viscosity $[\eta]$ > 2.5 m$^3$/kg and a concentration of < 0.2 % w/v.

2. Tear substitute according to claim 1, wherein the hyaluronan intrinsic viscosity $[\eta] \geq$ 2.9 m$^3$/kg.

3. Tear substitute according to claim 1 or 2, consisting of only substances naturally present in the human eye plus eventually substances which are pharmacologically, metabolically, immunologically and/or anti-microbial effective, or being preservative-free.

4. Tear substitute according to claim 1 or 2, containing glycosaminoglycans, electrolytes, and buffers, wherein preferably the electrolytes include sodium chloride, and/or the buffers include a phosphate buffer in concentration $\leq$ 1.45 mmol/l or trometamol.

5. Tear substitute according to claim 1 or 2, containing $\leq 2$ % w/v, preferably $\leq 0.1$ % w/v, and especially $\leq 0.01$ % w/v substances which are pharmacologically, metabolically, immunologically and/or anti-microbial effective.

6. Tear substitute according to any of the preceding claims, wherein the relation between average molecular mass and intrinsic viscosity $[\eta]$ in $m^3/kg$ is given through the equation

$$[\eta] \; = \; k \; \cdot \; (M_{rm})^a$$

with $M_{rm}$ being the average molecular mass in MDa and the coefficients

$$k = 1.3327 \cdot 10^{-4}$$

and

$$a = 0.6691.$$

7. Fluid, containing hyaluronan with an intrinsic viscosity $[\eta] > 2.5$ $m^3/kg$ and a concentration of $< 0.2$ % w/v, **characterized in that** said fluid being used as a tear substitute.

8. Fluid according to claim 7, wherein the hyaluronan intrinsic viscosity $[\eta] \geq 2.9$ $m^3/kg$.

9. Fluid according to claim 7 or 8, consisting of only substances naturally present in the human eye plus eventually substances which are pharmacologically, metabolically, immunologically and/or anti-microbial effective, or being preservative-free
and/or

10. Fluid according to claim 7 or 8, containing glycosaminoglycans, electrolytes, and buffers, wherein preferably the electrolytes include sodium chloride, and/or the buffers include a phosphate buffer in concentration $\leq 1.45$ mmol/l or trometamol.

11. Fluid according to claim 7 or 8, containing $\leq 2$ % w/v, preferably $\leq 0.1$ % w/v, and especially $\leq 0.01$ % w/v substances which are pharmacologically, metabolically, immunologically and/or anti-microbial effective.

12. Fluid according to any of claims 7 to 11, wherein the relation between average molecular mass and intrinsic viscosity $[\eta]$ in $m^3/kg$ is given through the equation

$$[\eta] \; = \; k \; \cdot \; (M_{rm})^a$$

with $M_{rm}$ being the average molecular mass in MDa and the coefficients

$$k = 1.3327 \cdot 10^{-4}$$

and

$$a = 0.6691.$$

13. Method of producing a tear substitute, comprising the use of hyaluronan with an intrinsic viscosity $[\eta] > 2.5$ $m^3/kg$ and a concentration of $< 0.2$ % w/v.

14. Method according to claim 13, wherein the hyaluronan intrinsic viscosity $[\eta] \geq 2.9$ $m^3/kg$.

15. Method according to claim 13 or 14, wherein substances which are increasing the viscosity are added towards or during or as a final step,

and/or

wherein mixing is carried out so as to reach or until reaching a homogeneous mixture,
and/or
wherein initially purified water or water for injection is provided for as a basis, wherein preferably electrolytes, buffers and substances which are not increasing the viscosity are added at first to the purified water or water for injection.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20040013729 A1